# EUROPÄISCHE PATENTSCHRIFT

(11) **EP 1 286 943 B1**
(45) Veröffentlichungstag und Bekanntmachung des Hinweises auf die Patenterteilung: **02.11.2011**
(21) Anmeldenummer: 01933973.8
(22) Anmeldetag: 16.05.2001
(51) Int. Cl.: C07C 51/43, C07C 57/04

(54) **VERFAHREN DER DISKONTINUIERLICHEN KRISTALLISATIVEN REINIGUNG VON ROH-ACRYLSÄURE**
METHOD FOR DISCONTINUOUS PURIFICATION OF CRUDE ACRYLIC ACID BY MEANS OF CRYSTALLIZATION
PROCEDE DE PURIFICATION DISCONTINUE D'ACIDE ACRYLIQUE BRUT PAR CRISTALLISATION

(30) Priorität: 29.05.2000 DE 10026407
(43) Veröffentlichungstag der Anmeldung: 05.03.2003
(73) Patentinhaber: BASF SE, 67056 Ludwigshafen (DE)
(72) Erfinder: ECK, Bernd, 68519 Viernheim (DE); BAUMANN, Dieter, 67227 Frankenthal (DE); HEILEK, Jörg, 69245 Bammental (DE); MÜLLER-ENGEL, Klaus, Joachim, 76297 Stutensee (DE)
(86) Internationale Anmeldenummer: PCT/EP2001/005551
(87) Internationale Veröffentlichungsnummer: WO 2001/092197

(56) Entgegenhaltungen:
- EP-A- 0 373 728
- DE-A- 19 829 477
- US-A- 4 882 430

## Beschreibung

Vorliegende Erfindung betrifft ein Verfahren zur diskontinuierlichen Reinigung von Roh-Acrylsäure, das wenigstens eine Abtriebsstufe und wenigstens eine Reinigungsstufe umfaßt.

Acrylsäure ist eine bedeutende Grundchemikalie, die z.B. als solche oder in Gestalt ihrer Alkylester zur Herstellung von radikalisch erzeugten Polymerisaten Verwendung findet.

Üblicherweise erfolgt die Herstellung von Acrylsäure durch heterogen katalysierte Gasphasenoxidation von Propen und/oder Propan (vgl. z.B. DE-A 19 508 558, EP-A 293 224, EP-A 257 565 und die in diesen Schriften zitierte Literatur).

Das die Gasphasenoxidation verlassende Gasgemisch besteht selbstredend nicht aus reiner Acrylsäure sondern aus einem letztere enthaltenden Gasgemisch, aus welchem Acrylsäure abgetrennt werden muß.

Die verschiedenen bekannten Varianten der Acrylsäureabtrennung aus dem Produktgasgemisch der Gasphasenoxidation sind z.B. in der DE-A 19 600 955 zusammenfassend dargestellt.

Gemeinsames Merkmal der bekannten Trennverfahren ist, daß die gewünschte Acrylsäure zunächst entweder durch Absorption mit einem Lösungsmittel (vgl. auch DE-A 4 308 087) oder durch partielle Kondensation aus dem Reaktionsgasgemisch abgetrennt wird. Das dabei resultierende Absorbat bzw. Kondensat wird anschließend in der Regel destillativ (gegebenenfalls unter Zusatz eines azeotropen Schleppmittels) und/oder exktraktiv aufgearbeitet und so eine Acrylsäurequalität gewonnen, die normalerweise, bezogen auf ihr Gewicht, ≥ 95 Gew. -%, häufig ≥ 96 Gew. -% oder ≥ 97 Gew. -%, vielfach ≥ 98 Gew. -%, teilweise ≥ 99 Gew. -% und teilweise sogar ≥ 99,5 Gew. -% an Acrylsäure enthält.

Acrylsäuren der vorgenannten Qualitäten sollen in dieser Schrift ganz allgemein unter der Bezeichnung "Roh-Acrylsäure" zusammengefasst werden. In typischer Weise enthalten sie wenigstens eine der nachfolgenden Verunreinigungen: Wasser, Essigsäure, Propionsäure, niedermolekulare Aldehyde wie Acrolein, Furfural, Benzaldehyd, Ester aus Acrylsäure und Allylalkohol, Maleinsäureanhydrid, Prozeßpolymerisationsinhibitor wie z.B. Phenothiazin und/oder N-Oxyl-Radikale sowie Diacrylsäure (das durch Michael-Addition von Acrylsäure an sich selbst beim sich selbst Überlassen von Acrylsäure enthaltenden Gemischen entstehende Addukt).

Bei der Verwendung von Acrylsäure für z.B. radikalische Polymerisationszwecke erweist sich die Mehrzahl der vorgenannten Verunreinigungen als störend (z.B. bewirken sie Verfärbungen der resultierenden Polymerisate oder beeinflussen die Induktionszeit, das ist die Zeitdauer bis zum Beginn der Polymerisation, in nachteiliger Weise).

Als besonders unerwünschte Verunreinigung erweist sich dabei Diacrylsäure. Sie wird zwar in der Regel radikalisch copolymerisiert, das jedoch mit dem Nachteil, daß sie bei thermischer Belastung des Polymerisats rückspaltet und dabei monomere Acrylsäure freisetzt, was insbesondere bei Verwendung des Polymerisats im Hygienebereich (Stichwort: Superabsorber in Babywindeln; bei diesen Polymerisaten handelt es sich um teilneutralisierte, radikalisch polymerisierte Polyacrylsäuren) nicht tolerabel ist.

Im Normalfall findet daher Roh-Acrylsäure nicht als solche, sondern erst als in zu Rein-Acrylsäure weitergereinigter Form Verwendung.

In der Literatur (vgl. z.B. EP-A 616 998) wird vorgeschlagen, die Reinigung von Roh-Acrylsäure zu Rein-Acrylsäure durch diskontinuierliche Kristallisation durchzuführen. Dabei wird die Ausgangs-Roh-Acrylsäure durch Einwirkung von Kälte in einem ersten Kristallisationsschritt in, eine höhere Reinheit als die Ausgangs-Roh-Acrylsäure aufweisende Acrylsäurekristalle sowie in eine, eine geringere Reinheit als die Ausgangs-Roh-Acrylsäure aufweisende Mutterlauge aufgetrennt. Die Kristallisation kann dabei sowohl statisch (die Schmelze ruht während der Kristallisation, z.B. plattenkristallisator oder Rippenrohrkristallisator) als auch dynamisch (die Schmelze wird während der Kristallisation bewegt, z.B. Fallfilmkristallisator oder volldurchströmtes Rohr) durchgeführt werden. Die anfallenden Acrylsäurekristalle werden aufgeschmolzen und bei Bedarf in einem oder mehreren weiteren zeitlich aufeinanderfolgenden Kristallisationsschritten weitergereinigt.

Um die Ausbeute wirtschaftlich zu gestalten, wird auch die im ersten Kristallisationsschritt anfallende Mutterlauge wenigstens einem weiteren Kristallisationsschritt unterworfen. Die Kristallisationsschritte, bei denen die zu kristallisierende Schmelze aus den im ersten Kristallisationsschritt angefallenen Acrylsäurekristallen erwächst, werden generell als Reinigungsstufen bezeichnet. Ebenso wird der erste Kristallisationsschritt als Reinigungsstufe bezeichnet. Im Unterschied dazu werden alle Kristallisationsschritte, bei denen die zu kristallisierende Schmelze aus der im ersten Kristallisationsschritt angefallenen Mutterlauge erwachsen ist, als Abtriebsstufen bezeichnet.

D.h. eine diskontinuierliche kristallisative Reinigung von Roh-Acrlysäure umfaßt im Normalfall wenigstens eine Reinigungsstufe und wenigstens eine Abtriebsstufe.

Im Stand der Technik (z.B. EP-A 616 998) wird nun auch für die diskontinuierliche Reinigung von Roh-Acrylsäure empfohlen, sowohl die wenigstens eine Reinigungsstufe (d.h., auch alle gegebenenfalls zusätzlich angewandten Reinigungsstufen) als auch wenigstens die erste Abtriebsstufe in ein und demselben, d.h. in einem einzigen Kristallisator durchzuführen. Nachteilig an dieser Verfahrensweise ist, daß diejenigen Anteile der Ausgangs-Roh-Acrylsäure, die gerade nicht im Kristallisator einer Kristallisation unterworfen sind, in Behältern zwischengelagert werden. Während dieser Zwischenlagerung erfolgt jedoch Diacrylsäurebildung. In einer nachfolgenden Kristallisationsstufe wird die während der Zwischenlagerung gebildete Diacrylsäure zwar abgetrennt, doch ist die abgetrennte Diacrylsäure gleichbedeutend mit einer geringeren Ausbeute an Rein-Acrylsäure.

Aufgabe der vorliegenden Erfindung war es, ein verbessertes Verfahren der diskontinuierlichen kristallisativen Reinigung von Roh-Acrylsäure zur Verfügung zu stellen.

Dementsprechend wurde ein wenigstens eine Reinigungsstufe und wenigstens eine Abtriebsstufe umfassendes diskontinuierliches Verfahren zur Reinigung von Roh-Acrylsäure gefunden, das dadurch gekennzeichnet ist, daß wenigstens die erste Abtriebsstufe in einem anderen Kristallisator durchgeführt wird, als die erste Reinigungsstufe.

Vorliegende Erfindung ist vor allem insofern von Bedeutung, als die nach der ersten Reinigungsstufe verbleibende Menge an Mutterlauge wesentlich kleiner ist, als die in der ersten Reinigungsstufe kristallisativ zu reinigende Menge an Ausgangs-Roh-Acrylsäure.

Da sich die Dimensionierung des für die erste Reinigungsstufe verwendeten Kristallistors (nachfolgend Kristallisator A genannt) aber in notwendiger Weise an der zu dieser Stufe kristallisativ zu behandelnden Menge an Ausgangs-Roh-Acrylsäure orientieren muß, ist der Kristallisator A für eine kristallisative Weiterreinigung der zu der ersten Reinigungsstufe anfallenden Mutterlauge normalerweise überdimensioniert, weshalb in der Praxis, bei Verwendung lediglich eines einzigen Kristallisators für eine diskontinuierliche kristallisative Reinigung von Ausgangs-Roh-Acrylsäure, so verfahren wird, daß man mehrere in zeitlich nacheinander durchgeführten ersten Reinigungsstufen angefallene Mutterlaugen sammelt, bis die kristallisativ weiterzureinigende Menge an aus ersten Reinigungsstufen stammender Mutterlauge der Dimension des Kristallisators A angemessen ist (andernfalls ist die mit einem Kristallisator A erzielbare Raum-Zeit-Ausbeute an Rein-Acrylsäure nicht voll befriedigend).

Im vorgenannten Fall ist die Diacrylsäurebildung infolge der längeren erforderlichen Zeit zur Zwischenlagerung von Erstreinigungsstufen-Mutterlauge in letzterer besonders ausgeprägt.

Es ist daher erfindungsgemäß zweckmäßig, für die erste Abtriebsstufe einen Kristallisator einzusetzen (nachfolgend Kristallisator B genannt), der für eine kleinere zu kristallisierende Menge ausgelegt ist, die nur 10 bis 60 Vol. -%, vorzugsweise 20 bis 50 Vol. -%, besonders bevorzugt 20 bis 40 Vol. -% und ganz besonders bevorzugt 25 bis 35 Vol. -% der in der ersten Reinigungsstufe kristallisativ zu reinigenden Menge an Ausgangs-Roh-Acrylsäure beträgt.

Erfindungsgemäß kann sowohl der Kristallisator A als auch der Kristallisator B ein dynamischer oder ein statischer Kristallisator sein. Selbstverständlich kann aber auch der Kristallisator A ein dynamischer und der Kristallisator B ein statischer Kristallisator (oder umgekehrt) sein.

Erfingdungsgemäß bevorzugt ist es, wenn sowohl der Kristallisator A als auch der Kristallisator B ein dynamischer Kristallisator ist.

Besonders zweckmäßig ist es, wenn es sich sowohl beim Kristallisator A als auch beim Kristallisator B um Fallfilmkristallisatoren, mit Vorteil in beiden Fällen um Röhren-Fallfilm-Kristallisatoren handelt, wie sie in der EP-A 616 998 beschrieben sind.

In diesem Fall enthält ein erfindungsgemäß verwendeter Röhren-Fallfilm-Kristallisator B mit Vorteil lediglich 10 bis 60 %, bevorzugt 20 bis 50 %, besonders bevorzugt 20 bis 40 % und ganz besonders bevorzugt 25 bis 35 % der im erfindungsgemäß zu verwendenden Röhren-Fallfilm-Kristallisator A enthaltenen Kristallisationsrohre.

Im übrigen können die erfindungsgemäß zu verwendenden wenigstens zwei Kristallisatoren A und B in an sich bekannter Weise betrieben werden.

Eine erfindungsgemäße Paarung aus Kristallisatoren A und B soll für die Zwecke dieser Erfindung als Kristallisator-Tandem bezeichnet werden.

Abschließend sei festgehalten, daß das erfindungsgemäße Verfahren aber auch dann in hervorragender Weise Anwendung findet, wenn die in der ersten Reinigungsstufe ausgefrorene Acrylsäuremenge nur bis zu 30 Gew. -% der eingesetzten Ausgangs-Roh-Acrylsäure beträgt.

Selbstverständlich können der Kristallisator A erfindungsgemäß für weitere Reinigungsstufen (zweite, dritte etc.) und der Kristallisator B für weitere Abtriebsstufen (zweite, dritte etc.) eingesetzt werden.

Das erfindungsgemäße Verfahren ist bei gleicher Produktionskapazität an Rein-Acrylsäure bezüglich des Abfalls an Diacrylsäure immer günstiger als ein Verfahren, das lediglich einen Kristallisator einsetzt.

Seine Vorteilhaftigkeit kommt insbesondere dann zum Tragen, wenn sowohl mehr als eine Reinigungsstufe als auch mehr als eine Abtriebsstufe angewendet werden.

## Patentansprüche

1. Verfahren der diskontinuierlichen kristallisativen Reinigung von Roh-Ac.rylsäure, die ≥ 95 Gew. -% an Acrylsäure enthält, umfassend wenigstens eine Reinigungsstufe und wenigstens eine Abtriebsstufe, **dadurch gekennzeichnet, daß** wenigstens die erste Abtriebsstufe in einem anderen Kristallisator, in einem Kristallisator B, durchgeführt wird als die erste Reinigungsstufe, die in einem Kristallisator A durchgeführt wird, wobei die in der ersten Abtriebsstufe kristallisierte Schmelze in der ersten-Reinigungsstufe angefallene Mutterlauge ist.

2. Verfahren nach Anspruch 1, **dadurch gekennzeichnet, daß** sowohl der Kristallisator A als auch der Kristallisator B ein dynamischer Kristallisator ist.

3. Verfahren nach Anspruch 1, **dadurch gekennzeichnet, daß** sowohl der Kristallisator A als auch der Kristallisator B ein Röhren-Fallfilm-Kristallisator ist.

4. Verfahren nach einem der Ansprüche 1 bis 3, **dadurch gekennzeichnet, daß** die zu reinigende Menge, für die der Kristallisator B ausgelegt ist, lediglich 10 bis 60 Vol. -% der zu reinigenden Menge beträgt, für die der Kristallisator A ausgelegt ist.

5. Verfahren nach Anspruch 3, **dadurch gekennzeichnet, daß** der Röhren-Fallfilm-Kristallisator B lediglich 10 bis 60 % der Anzahl an Kristallisationsröhren des Röhren-Fallfilm-Kristallisators A enthält.

## Claims

1. A process for the batchwise purification of crude acrylic acid which comprises ≥ 95% by weight of acrylic acid by crystallization, comprising at least one purification stage and at least one stripping stage, wherein at least the first stripping stage is carried out in a different crystallizer, in a crystallizer B, from the first purification stage, which is carried out in a crystallizer A, the melt crystallized in the first stripping stage being mother liquor produced in the first purification stage.

2. The process according to claim 1, wherein both the crystallizer A and the crystallizer B are dynamic crystallizers.

3. The process according to claim 1, wherein both the crystallizer A and the crystallizer B are tubular falling-film crystallizers.

4. The process according to any of claims 1 to 3, wherein the amount to be purified, for which the crystallizer B is designed, is only from 10 to 60% by volume of the amount to be purified for which the crystallizer A is designed.

5. The process according to claim 3, wherein the tubular falling-film crystallizer B comprises only from 10 to 60% of the number of crystallization tubes of the tubular falling-film crystallizer A.

## Revendications

1. Procédé de purification par cristallisation discontinue d'acide acrylique brut qui contient ≥ 95 % en poids d'acide acrylique, comprenant au moins une étape de purification et au moins une étape de rectification, **caractérisé en ce qu'**au moins la première étape de rectification est effectuée dans un autre cristalliseur, dans un cristalliser B, que la première étape de purification, qui est effectuée dans un cristallisent A, la masse fondue cristallisée dans la première étape de rectification étant la liqueur-amère formée dans à première étape de purification.

2. Procédé selon la revendication 1, **caractérisé en ce qu'**aussi bien le cristalliser A que le cristalliseur B est un cristalliseur dynamique.

3. Procédé selon la revendication 1, **caractérisé en ce qu'**aussi bien le cristalliseur A que le cristalliseur B est un cristalliseur à tubes à film descendant.

4. Procédé selon l'une quelconque des revendications 1 à 3, **caractérisé en ce que** la quantité à purifier, pour laquelle est conçu le cristalliseur B, ne représenté que 10 à 60 % en volume de la quantité à purifier pour laquelle est conçu le cristalliseur A.

5. Procédé selon la revendication 3, **caractérisé en ce que** le cristalliseur B à tubes à film descendant ne contient que 10 à 60 % du nombre de tubes de cristallisation du cristalliseur à tubes à film descendant A.
